(19) Europäisches Patentamt
European Patent Office
Office européen des brevets

(11) EP 4 371 481 A1

(12) EUROPEAN PATENT APPLICATION

(43) Date of publication:
22.05.2024 Bulletin 2024/21

(51) International Patent Classification (IPC):
$A61B\ 5/145^{(2006.01)}$   $A61B\ 5/083^{(2006.01)}$

(21) Application number: 22208583.9

(52) Cooperative Patent Classification (CPC):
A61B 5/0836; A61B 5/14542

(22) Date of filing: 21.11.2022

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: Lumetry Diagnostics GmbH
8020 Graz (AT)

(72) Inventors:
• NEUBAUER, Christian
  8020 Graz (AT)
• RÜTHER, Horst
  8020 Graz (AT)
• SORANTIN, Max
  8020 Graz (AT)

(74) Representative: SONN Patentanwälte GmbH & Co
KG
Riemergasse 14
1010 Wien (AT)

(54) **METHOD AND APPARATUS FOR DETERMINING ARTERIAL CO2 CONCENTRATION**

(57)    The present invention provides a method for determining an arterial $CO_2$ concentration ($PaCO_2$) level in a subject, the method comprising the following steps:
- measuring $CO_2$ concentration levels in exhaled breath from the subject over a period of time in order to obtain a capnography signal;
- deriving a $CO_2$ waveform signal from the capnography signal;

- determining an End-tidal $CO_2$ ($EtCO_2$) value from the $CO_2$ waveform signal;
- analyzing a shape of the $CO_2$ waveform signal in order to determine a $CO_2$ mismatch correction factor;
- applying the $CO_2$ mismatch correction factor to the $EtCO_2$ value in order to obtain the $PaCO_2$ level.

    The present invention further provides an apparatus for determining a $PaCO_2$ concentration level.

Fig.1

**Description**

The field of the present invention is the field of arterial carbon dioxide ($CO_2$) concentration measurement.

In clinical diagnostics, arterial blood gas analysis measures the amounts of arterial gases in a blood sample taken from a subject's artery. Such analyses are used in various areas of medicine, e.g. emergency medicine or pulmonology. Typically, arterial blood gas analysis includes measurements of the concentration of oxygen (arterial partial pressure of oxygen; $PaO_2$) and the concentration $CO_2$ (arterial partial pressure of $CO_2$; $PaCO_2$).

$PaCO_2$ levels are of particular medical interest. For instance, in patients with hypercapnia, the arterial $CO_2$ partial pressure can reach critical values within just a few days, requiring immediate clinical admission. Since arterial blood gas measurements are not readily available at patients' homes, there is an urgent need for easily accessible low-cost tools to determine $PaCO_2$ levels without trained personnel.

Respiratory gas analysis has the potential to serve as such a tool. Capnometers and Capnographs measure the concentration of $CO_2$ in expired air. They typically use infrared measurement to detect the torsional and/or stretching vibration of the $CO_2$ molecule in order to measure the $CO_2$ concentration. "Capnometry" is typically used as a general term to refer to the measurement of $CO_2$ concentrations in respiratory gas. The term "Capnography" typically refers more specifically to the continuous analysis and recording of the $CO_2$ concentration over a certain period of time. Capnographs typically present the measured data as a series of waveforms representing the $CO_2$ concentration in the breath as a function of time, e.g., on a display integrated in the capnograph device.

Capnographs are used to assess a patient's respiratory status. Typically, the so-called end-tidal carbon dioxide ($EtCO_2$) is measured, which is the level of $CO_2$ at the end of an exhaled breath.

The $EtCO_2$ (end-tidal $CO_2$ in exhaled breath) and the $PaCO_2$ (arterial $CO_2$) are related, but not the same. The difference between $EtCO_2$ and $PaCO_2$ is commonly referred to as "mismatch". Typical mismatch values are known; thus, it is to some extent possible to draw conclusions about arterial blood partial pressures from capnometric measurements. However, such estimation of $PaCO_2$ from capnometric measurements lacks accuracy and reliability. This is in particular true for individuals with changes in lung tissue and/or emphysema, where mismatch values can be entirely different from healthy individuals (but this mismatch can only be used as a diagnostic parameter, if $PaCO_2$ values from blood are available). In many cases capnometric measurements in sick individuals will significantly under- or overestimate $PaCO_2$ levels, thereby giving the patient a false sense of security.

Thus, easily accessible yet reliable tools for determining $PaCO_2$ levels, in particular determining $PaCO_2$ levels from the breath, especially in sick individuals, are still lacking. It is an object of the invention to provide such tools.

This object is solved by the method and apparatus as defined in the independent claims.

In a first aspect, the invention provides a method for determining an arterial $CO_2$ concentration ($PaCO_2$) level in a subject, the method comprising the following steps:

- measuring $CO_2$ concentration levels in exhaled breath from the subject over a period of time in order to obtain a capnography signal;
- deriving a $CO_2$ waveform signal from the capnography signal;
- determining an End-tidal $CO_2$ ($EtCO_2$) value from the $CO_2$ waveform signal;
- analyzing a shape of the $CO_2$ waveform signal in order to determine a $CO_2$ mismatch correction factor;
- applying the $CO_2$ mismatch correction factor to the $EtCO_2$ value in order to obtain the $PaCO_2$ level.

In a further aspect, the invention provides an apparatus for determining a $PaCO_2$ concentration level in a subject, the apparatus comprising:

- at least one capnography sensor configured to measure $CO_2$ concentration levels in exhaled breath from the subject as a function of time;
- a processor configured to carry out the following steps:

  o obtaining from the capnography sensor a capnography signal comprising $CO_2$ concentration levels in exhaled breath from the subject over a period of time;
  o deriving a $CO_2$ waveform signal from the capnography signal;
  o determining an End-tidal $CO_2$ ($EtCO_2$) value from the $CO_2$ waveform signal;
  o analyzing a shape of the $CO_2$ waveform signal in order to determine a $CO_2$ mismatch correction factor;
  o applying the $CO_2$ mismatch correction factor to the $EtCO_2$ value in order to obtain the $PaCO_2$ level.

Preferably, the processor of the inventive apparatus is configured to carry out the steps as defined with respect to the inventive method. Thus, herein all detailed descriptions of the inventive method equally apply to the apparatus of the invention and vice versa.

The invention is based on the surprising finding, that the mismatch between the arterial $PaCO_2$ value and the respiratory $EtCO_2$ is correlated to the shape of the waveform of $CO_2$ concentration in exhaled air. Thus, it was surprisingly found that by analyzing the shape of a capnographic curve, it is possible to estimate the mismatch and thus the $PaCO_2$ level. This is of special importance for subjects with medical conditions, for whom the mismatch is often particularly large. However, also for healthy subjects, the inventive method allows determining $PaCO_2$ more precisely.

Already prior to the invention it was known that certain disease states can influence the shape of a capnographic curve. For instance, EP 2 438 855 A2 relates to methods for interpreting capnographic waveforms. A first aspect disclosed therein relates to a method for diagnosis of a respiratory status of a patient, comprising analyzing a shape of a recorded $CO_2$ waveform; extracting, from the analyzed shape, at least one parameter by which the shape is characterized; and generating a diagnosis of the respiratory status of the patient, based on the at least one parameter. The parameter, on which the diagnosis is based, may e.g. be one or more of an angle ($\alpha$) between an alveolar rise and an alveolar plateau of the waveform, an angle ($\beta$) between the alveolar plateau and a descending limb of the waveform, $EtCO_2$, a final inspired $CO_2$ level ($FiCO_2$), or an overall rate of rise of $CO_2$. For instance, with respect to the angle $\alpha$, EP 2 438 855 A2 teaches that said angle is determined primarily by the V/Q (ventilation/perfusion) status of the lungs, and that patients with obstructions of the airway, such as in the case of chronic obstructive pulmonary disease (COPD) or asthma, have an increased $\alpha$ angle. According to EP 2 438 855 A2, the $\alpha$ angle is thus a widely used parameter for a first-hand assessment of the patient's overall pulmonary state.

Thus, it was known that the shape of the capnographic curve is related to disease states of the patient. However, prior to the present invention it was entirely unexpected that the shape of the capnographic curve could also be used to estimate mismatch values between $EtCO_2$ and arterial $PaCO_2$. Just how unexpected this finding of the present inventors was, is shown by the fact that the above-mentioned EP 2 438 855 A2 in a second aspect also set out to provide a method for determining arterial $PaCO_2$ values. EP 2 438 855 A2 states in paragraph [0010] that "the need to be able to determine the true value of $PaCO_2$ from the measured value of $EtCO_2$ is of great importance". In the same paragraph it is noted that the value of $PaCO_2$ is close to that of $EtCO_2$ only in subjects in good respiratory health, but that for subjects with any form of dead space ventilation, or with defective perfusion mechanisms, the two values can be widely different. The authors of EP 2 438 855 A2 attempted to solve this problem by measuring the partial pressure of oxygen in the patient's breath in addition to and simultaneously with $CO_2$. The obtained oxygen level is then used as an indication of the perfusion efficiency to provide an indication of discrepancy between the values of $EtCO_2$ obtained from the $CO_2$ capnographic values, and the value of the arterial $PaCO_2$ (EP 2 438 855 A2, paragraph [0011]). In addition to the determination of oxygen utilization, EP 2 438 855 A2 proposes to use a flow meter sensor to measure the ventilated volume of gas, which may additionally be used for estimating the discrepancy between the measured values of $EtCO_2$ and $PaCO_2$.

It is important to point out that the authors of EP 2 438 855 A2 did not consider using the shape of the capnographic curve to estimate the mismatch between the $EtCO_2$ and the $PaCO_2$ values. This is despite the fact that, according to the first aspect described in EP 2 438 855 A2, the authors knew that the shape of the curve is altered in sick individuals. The fact that the authors nevertheless chose the much more costly and elaborate methods of measuring the $O_2$ concentration in the breath as well as the ventilated volume of gas in addition to $CO_2$ shows just how surprising it was that the capnographic waveform itself could be used to correct the $EtCO_2$ value.

The finding by the present inventors, that the shape of the capnographic waveform itself can be used to estimate arterial $PaCO_2$ from respiratory $EtCO_2$, now makes it possible to dispense with costly additional measurements and sensors, such as for measuring $O_2$ and ventilated volume. Instead, the invention uses data that are already recorded in a normal capnographic measurement. Of course, the further sensors can be used in addition thereto, if an even more precise estimation of $PaCO_2$ is desired.

In the context of the inventive method, the $CO_2$ concentration levels in exhaled breath from the subject are preferably measured using a capnography sensor, preferably using the apparatus according to the invention. Advantageously, the inventive apparatus may be a portable and/or even a handheld capnograph.

The capnography signal that is obtained from the measuring of $CO_2$ concentration levels in exhaled breath typically is a capnogram. The capnography signal therefore preferably comprises $CO_2$ concentration levels as a function of time.

In an alternative embodiment of the invention, volumetric capnography may be used. In this case, the capnography signal preferably comprises $CO_2$ concentration levels as a function of exhaled volume. Expressing $CO_2$ concentration levels as function of exhaled volume can in certain cases allow even more precise estimation of mismatch and thus $PaCO_2$ levels.

In an embodiment, the $CO_2$ concentration levels are measured over a period of time corresponding to at least one breathing cycle, preferably at least two breathing cycles, more preferably at least five breathing cycles, most preferably at least ten breathing cycles. Extending the measurement over multiple breathing cycles allows to increase accuracy and precision since the influence of variations between individual breaths can be reduced. It is therefore advantageous when multiple repeated breathing cycles are measured. In the context of the invention, the term "breathing cycle" preferably corresponds to one full cycle consisting of one expiration and one inspiration.

It is further preferred if the $CO_2$ concentration levels are measured over a period of time of at least 5 seconds, preferably at least 10 seconds, more preferred at least 20 seconds, even more preferred at least 30 seconds, yet even more preferred at least 45 seconds, most preferred at least 60 seconds. Preferably, the $CO_2$ concentration levels are measured over a period of time of between 5 seconds and 15 minutes, preferably between 10 seconds and 10 minutes, more preferred between 20 seconds and 6 minutes, even more preferred between 30 seconds and 4 minutes, most preferred between 45 seconds and 2 minutes. Extending the measurement over such a period of time allows to increase the accuracy and precision of the measurement, for the same reasons as laid out above.

In an embodiment, the measuring of $CO_2$ concentrations is continued until a pre-determined reproducibility criterion is met. For instance, the measurement may be repeated until a certain number of successive measurements lie within a pre-determined window of tolerance.

In a preferred embodiment, the capnography signal contains at least one, preferably at least two, even more preferred at least five $CO_2$ concentration values per second. It is further preferred if the capnography signal contains at least 10, preferably at least 20, more preferably at least 40, even more preferably at least 100 $CO_2$ concentration values. This allows even more precise and accurate measurements.

In the context of the inventive method, the deriving of the $CO_2$ waveform signal may consist of extracting a pre-determined time-period from the capnography signal. For instance, the $CO_2$ waveform signal may correspond to a certain time interval from the capnography signal, e.g. a time interval corresponding to one full breathing cycle.

The $CO_2$ waveform signal may correspond to the $CO_2$ concentration as a function of time. In an alternative embodiment, the $CO_2$ waveform signal corresponds to the $CO_2$ concentration as a function of exhaled volume. Expressing $CO_2$ concentration levels as function of exhaled volume can in certain cases allow even more precise estimation of mismatch and thus $PaCO_2$ levels.

Preferably, the $CO_2$ waveform signal comprises at least one expiratory upstroke and one alveolar plateau. It was found that the shape of the waveform in the expiratory upstroke and the alveolar plateau is particularly informative with regards to the mismatch.

In an embodiment, the $CO_2$ waveform signal further comprises at least one respiratory baseline. This provides even more information to the estimation of the mismatch and thus allows for even more accurate estimates.

Optionally, the $CO_2$ waveform signal may further comprise an inspiratory downstroke (alternatively or in addition to the respiratory baseline). However, for the purposes of the inventive method it is also sufficient to consider the capnographic curve only until it reaches the end-tidal value (i.e., $EtCO_2$).

In an embodiment, deriving the $CO_2$ waveform signal from the capnography signal comprises detecting individual breaths in the capnography signal. Detecting individual breaths may, e.g., be done by generating a mean line within the capnography signal corresponding to a mean $CO_2$ concentration (or another $CO_2$ concentration between the maximum and the minimum) and locating the intersections of the capnography signal with the mean line. The intersections then correspond to the transitions from inhaling to exhaling and vice versa.

In an embodiment, the $CO_2$ waveform signal may be extracted from one detected breathing cycle in the capnography signal. Thus, the $CO_2$ waveform signal may correspond to the expiratory upstroke and the alveolar plateau (and, optionally, the respiratory baseline and/or the inspiratory downstroke) of a single breathing cycle.

In a further embodiment, the $CO_2$ waveform signal may be extracted from multiple breathing cycles in the capnography signal. In a preferred embodiment, the $CO_2$ waveform signal is extracted from at least two, preferably at least three, more preferred at least six, especially at least eight breathing cycles. Preferably, the $CO_2$ waveform signal corresponds to the average of at least two, preferably at least three, more preferred at least six, especially at least eight breathing cycles.

Obtaining the $CO_2$ waveform signal as the average over multiple breathing cycles can be achieved, e.g., as follows: The individual breathing cycles can be detected as described above. Next, the capnography signal can be subdivided into the individual consecutive breathing cycles. These individual breathing cycles can then be assembled into a master breathing curve by synchronizing them (e.g., by finding a synchronization point in which the concentration change from a point to the next for the first time exceeds a certain value) followed by averaging. From this master breathing curve, the $CO_2$ waveform signal may then be extracted, e.g., again comprising an expiratory upstroke and an alveolar phase and, optionally, a respiratory baseline and/or an inspiratory downstroke. In this case, the expiratory upstroke of the $CO_2$ waveform signal corresponds to the average of the expiratory upstrokes of multiple breathing cycles, the alveolar plateau of the $CO_2$ waveform signal corresponds to the average of the alveolar plateaus of multiple breathing cycles, etc. Taking the average over multiple breathing cycles allows to reduce the impact of variations between individual breath and thus improves precision and accuracy.

In a preferred embodiment, the deriving the $CO_2$ waveform signal comprises filtering out breathing cycles that do not fulfil at least one predetermined inclusion criterion. For instance, when the capnography signal comprises at least six breathing cycles, the breathing cycle having the largest average deviation from the average (the master breathing curve) may be filtered out. In this case, the $CO_2$ waveform signal may correspond to the average of the remaining breathing cycles.

Methods for determining the $EtCO_2$ value are known in the art, as this value is commonly measured with existing capnographs. In the context of the inventive method, $EtCO_2$ may simply be obtained be determining the maximum $CO_2$ concentration of the capnography signal and/or the maximum $CO_2$ concentration of the $CO_2$ waveform signal. Alternatively, $EtCO_2$ may be obtained by determining the $CO_2$ concentration at the end of a detected expiration. As a further alternative, the $CO_2$ waveform signal may also be fitted to a set of mathematical functions and the $EtCO_2$ value may be derived from these functions.

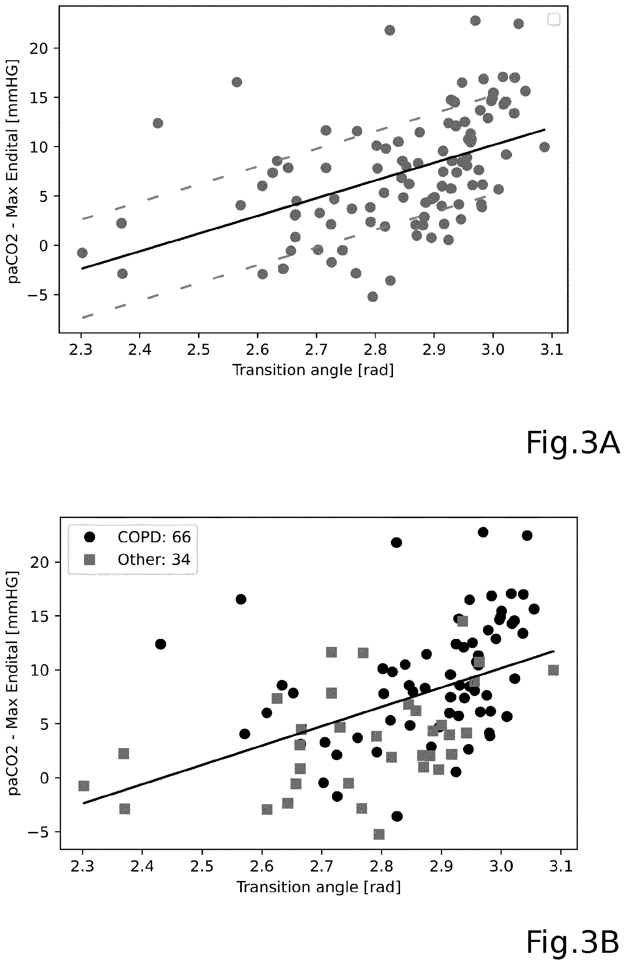

In the context of the invention, any suitable method for determining the $CO_2$ mismatch correction factor based on the shape of the $CO_2$ waveform signal may be used. In a preferred embodiment, the $CO_2$ mismatch correction factor is based on an angle ($\alpha$) between an expiratory upstroke and an alveolar plateau of the $CO_2$ waveform signal. In the context of the invention, it has been surprisingly found that this angle $\alpha$ strongly correlates with the mismatch between respiratory $EtCO_2$ and arterial $PaCO_2$ (see Example 2 and Figure 3). Thus, by correcting the measured $EtCO_2$ using the angle $\alpha$, a particularly accurate estimate of $PaCO_2$ can be obtained.

In a preferred embodiment, the analyzing of a shape of the $CO_2$ waveform signal comprises fitting an expiratory upstroke to a first linear function, fitting an alveolar plateau to a second linear function and determining an angle ($\alpha$) between the first linear function and the second linear function. The angle $\alpha$ can then be used to accurately estimate $PaCO_2$ based on measured $EtCO_2$.

In a further preferred embodiment, analyzing a shape of the $CO_2$ waveform signal comprises fitting a transition between an expiratory upstroke and an alveolar plateau to an exponential curve, wherein the $CO_2$ mismatch correction factor is based on a time constant ($\tau$) of the exponential curve. In this case, for example, the transition between the expiratory upstroke and the alveolar plateau may be fitted to the following function:

$$p\_CO_2(t) \ = \ p\_CO_{2,\infty}(1-e^{-(t/\tau)})-p\_CO_2(t=0.1)$$

Based on this fit the time constant $\tau$ can be determined and used for estimating $PaCO_2$ based on $EtCO_2$.

As another example, an artificial intelligence (AI) model may be used to determine the $CO_2$ mismatch correction factor based on the shape of the $CO_2$ waveform signal. In this case, the AI model may be trained using a dataset comprising both capnographic and direct blood gas measurements from healthy and/or sick individuals.

In the context of the invention the subject preferably is a human subject. In a preferred embodiment, the subject is an asthma patient and/or a Chronic Obstructive Pulmonary Disease (COPD) patient. It is further preferred, if the subject is a patient having pulmonary fibrosis. Such patients typically have increased mismatch between $EtCO_2$ and $PaCO_2$. It was surprisingly found that also in such sick patients with large mismatch values there is a strong correlation between the shape of the $CO_2$ waveform signal and the mismatch (see Example 2 and Figure 3B). Thus, the present invention advantageously allows to accurately estimate $PaCO_2$ levels in such patients.

In many cases it can be informative to monitor the mismatch between the arterial $PaCO_2$ value and the respiratory $EtCO_2$ over a certain period of time. An increase or a decrease in the mismatch value can be indicative of a change in lung function. This is of particular interest in patients, e.g., suffering from asthma, COPD and/or pulmonary fibrosis, in whom a change of the mismatch value over time can be a critical parameter for monitoring disease progression. Thus, in a preferred embodiment, the inventive method is for monitoring mismatch values in the subject, wherein the determination of the $PaCO_2$ level is performed at least 2 times, preferably at least 4 times, more preferably at least 6 times, even more preferably at least 10 times over a time period of at least 1 week, preferably at least 2 weeks, more preferably at least 4 weeks, most preferably at least 8 weeks.

In the context of the inventive method, the measuring of $CO_2$ concentrations in exhaled breath can be done using any suitable method known to the skilled person. Advantageously, an infrared (IR) measurement method may be used. Such methods can provide reliable measurements of $CO_2$ concentrations. Thus, in a preferred embodiment of the inventive apparatus, the capnography sensor is an IR-based capnography sensor.

In an embodiment, the apparatus comprises a sample chamber for taking up exhaled breath from the subject. Preferably, the sample chamber is temperature-controlled. Providing a temperature-controlled sample chamber allows to reduce condensation and, in this way, increases measurement accuracy.

In an embodiment, the apparatus further comprises a flow sensor. The flow sensor may be configured to measure the volume of exhaled breath passing through the sample chamber. Providing such a flow sensor allows to record the capnography signal as a function of exhaled volume. In this case, the measured $CO_2$ concentrations can be scaled to the measured breath volume of the flow sensor. Thus, this embodiment is particularly preferred when the capnography signal and/or the $CO_2$ waveform signal corresponds to the $CO_2$ concentration as a function of exhaled volume. However, the data obtained from a flow sensor can also improve measurement accuracy when time capnography is used, i.e., when the $CO_2$ waveform signal corresponds to the $CO_2$ concentration as a function of time. For instance, recording the flow allows to filter out breathing cycles with insufficient flow rates, thus further improving measurement accuracy. Any suitable type of flow sensor may be used, e.g., flow sensors based on propellors, anemometer, differential pressure, or ultrasound.

Optionally, the inventive method can also comprise the measurement of $O_2$ partial pressure in the exhaled breath. $O_2$ levels may, e.g., be determined using fluorescence-based sensors. Thus, the inventive apparatus may also include an $O_2$ concentration sensor, preferably a fluorescence-based $O_2$ sensor. Measuring $O_2$ concentrations allows to gain additional information about lung function and serves for extended diagnostic analysis.

However, in contrast to methods disclosed in the state of art, $O_2$ measurements or measurements of other gases are not required for determining $PaCO_2$ levels. Thus, in a preferred embodiment, the inventive method does not comprise determining the concentration levels of other gases in exhaled breath, especially wherein the method does not comprise determining oxygen concentration levels. Similarly, it is preferred that the inventive apparatus does not comprise further sensors for determining the concentration levels of other gases in exhaled breath, especially sensors for determining oxygen concentration levels.

In a preferred embodiment, the sample chamber comprises a hydrophilic or a hydrophobic coating. Such coatings allow reducing interferences and can further increase measurement accuracy.

Preferably, the apparatus comprises a temperature sensor and/or a humidity sensor configured to measure the temperature and/or humidity of the exhaled breath. Preferably these sensors are located in the sample chamber of the apparatus. Such sensors allow making corrections with respect to differences in vapor partial pressure, which can influence the accuracy of measurements in certain circumstances. Thus, the inventive method preferably comprises measuring the temperature and/or humidity in the exhaled breath, in order to correct the measured $CO_2$ concentration levels. Similarly, the processor of the inventive apparatus is preferably configured to correct the $CO_2$ concentration levels obtained from the capnography sensor based on the measured temperature and/or humidity.

In a further embodiment, the apparatus comprises a dichroic or semitransparent mirror, preferably in the sample chamber. Such a mirror allows detecting condensation and other interference optically.

In a preferred embodiment, the apparatus further comprises a mouth piece. A mouth piece allows to deliver breath to the apparatus in a particularly convenient manner. Alternatively, the breath may also be delivered to the apparatus through a breathing mask or through a nasal probe. Advantageously, the mouth piece, the breathing mask and/or the nasal probe may comprise an antimicrobial, especially an antibacterial and/or antiviral, coating and/or filter.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

Unless specified otherwise, the term "$CO_2$ concentration levels" or similar terms as used herein refers to the partial pressure of $CO_2$.

Unless specified otherwise, all parameters as used herein correspond to parameters at a temperature of 33 °C and a pressure of 101.300 Pa.

The present invention is further illustrated by the following figures and examples, without being limited thereto.

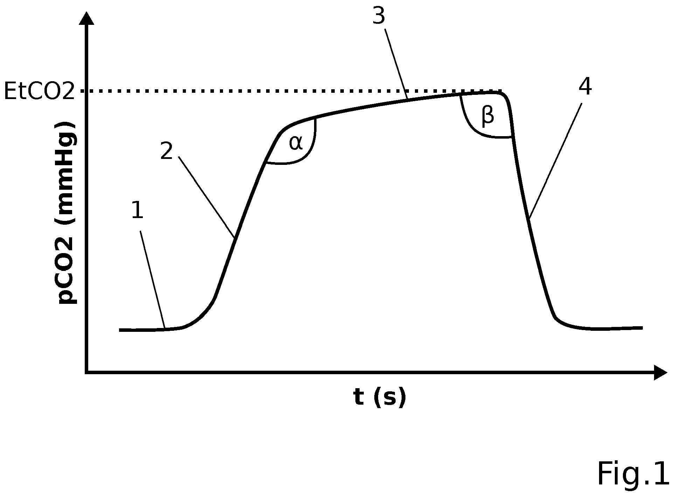

**Figure 1.** Schematic depiction of a $CO_2$ waveform signal corresponding to one respiration cycle of a capnographic signal. The graph displays $CO_2$ partial pressure (pCO2; e.g., measured in millimeter of mercury, mmHg) on the y-axis and time (t; e.g., measured in seconds, s) on the x-axis. The depicted $CO_2$ waveform signal comprises a respiratory baseline 1, an expiratory upstroke 2 (also referred to as alveolar rise), an alveolar plateau 3, and an inspiratory downstroke 4. Also displayed are the angle α between the expiratory upstroke 2 and the alveolar plateau 3, as well as the angle β between the alveolar plateau 3 and the inspiratory downstroke 4. The end-tidal carbon dioxide (EtCO₂) is displayed as the maximum $CO_2$ concentration of the $CO_2$ waveform signal.

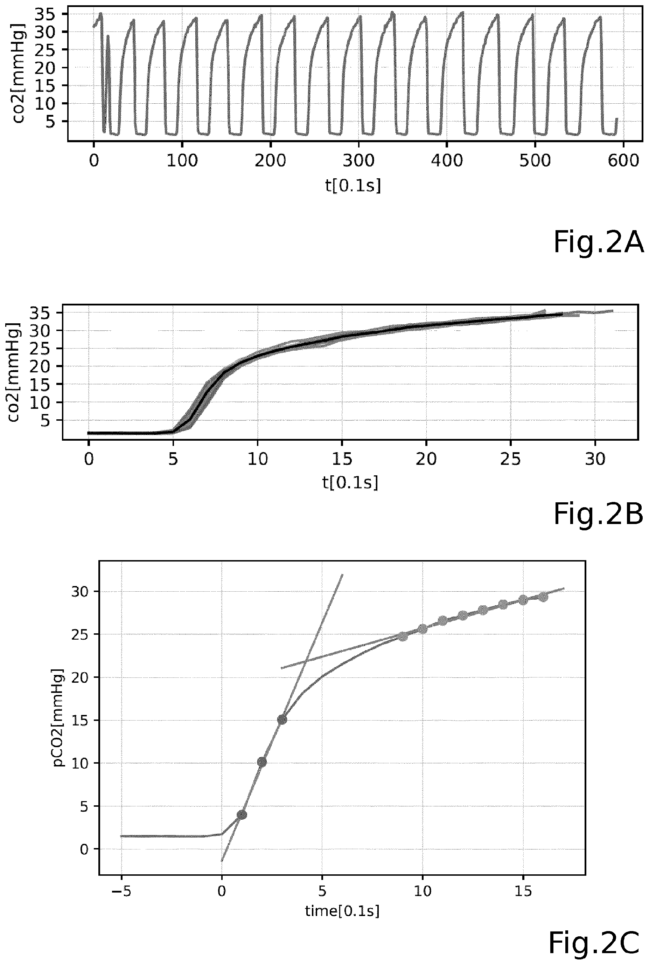

**Figure 2.** Determination of a $PaCO_2$ level according to an exemplary embodiment of the present invention. (A) Capnography signal obtained from a human adult subject. (B) Overlay of sections from the capnography signal corresponding to 14 synchronized breathing cycles extracted from the capnography signal. (C) $CO_2$ waveform signal corresponding to the mean of the overlaid curves shown in panel A. The expiratory upstroke and the alveolar plateau were each fitted to a linear function and the angle α between these lines was calculated.

**Figure 3.** Results from a correlation study involving 34 healthy subjects and 66 patients diagnosed with COPD. The mismatch between $PaCO_2$ determined from blood samples and EtCO₂ determined by capnography is displayed on the y-axis. The angle α obtained from the capnography measurements is displayed on the x-axis. (A) Graph including all subjects of the correlation study. (B) Same graph, wherein healthy subjects ("Other") and COPD patients are displayed individually.

**Example 1. Determination of a PaCO$_2$ level in a subject**

The present example describes the determination of a PaCO$_2$ level in a subject according to an exemplary embodiment of the invention.

Recording of capnography signal

A capnography signal was obtained from a human adult subject using a capnograph. Specifically, a mainstream capnograph with a nondispersive infrared spectrometer having a CO$_2$ sensor with a wavelength of 4.26 micrometers and a reference sensor at 3.91 micrometers was used. The subject was allowed to breathe through the mouthpiece of the capnograph for a time period of 1 minute. The CO$_2$ content in the breath was determined by analyzing the absorption of light in the CO$_2$ oscillation spectrum compared to calibration data. The CO$_2$ concentration (CO$_2$ partial pressure in mmHg) was recorded with a time resolution of 10 Hz, corresponding to 600 measurements per minute. The obtained capnography signal is shown in Figure 2A.

Detection of breathing cycles

For detecting individual breaths in the obtained capnography signal, a mean line was generated, corresponding to the mean CO$_2$ concentration in the capnography signal. The intersections were then taken as the transitions from inhaling to exhaling and vice versa. In total, 14 breathing cycles were detected.

Generation of a master breathing curve

The capnography signal was separated into 14 sections corresponding to the individual breathing cycles detected as described above. For each section, an EtCO$_2$ value corresponding to the maximum detected CO$_2$ concentration was determined. In the context of the present example, only the data up to the EtCO$_2$ were used; thus, the data points following the EtCO$_2$ value were deleted from each section.

Next, a synchronization point within each section was identified as the first data point in which the CO$_2$ concentration change with respect to the previous data point exceeded 1 mmHg. The sections were then synchronized based on the synchronization point and overlayed. The resulting overlay of the 14 synchronized sections is shown in Figure 2B.

In order to obtain a master breathing curve, the mean of the 14 synchronized sections was taken (i.e., at each time point the mean of the 14 CO$_2$ concentrations was calculated). The resulting master breathing curve was used as the CO$_2$ waveform signal for further analysis.

Deriving parameters from the CO$_2$ waveform signal

The EtCO$_2$ value was obtained by determining the maximum CO$_2$ concentration of each of the 14 sections used to obtain the master breathing curve and calculating the average of the 14 maxima obtained.

Next, the shape of the CO$_2$ waveform signal was analyzed. In the present example, the angle $\alpha$ between the expiratory upstroke 2 and the alveolar plateau 3 was used as a characteristic parameter of the shape in order to determine a CO$_2$ mismatch correction factor. To determine the angle, the datapoints of the CO$_2$ waveform signal corresponding to the expiratory upstroke 2 and the alveolar plateau 3 were determined. Next, the datapoints of the two sections were fitted to two linear functions and the angle between the two linear functions was calculated. This is shown in Figure 2C.

Calculation of PaCO$_2$

The PaCO$_2$ level was calculated by applying the CO$_2$ mismatch correction factor based on the angle $\alpha$ to the obtained EtCO$_2$ value. For this purpose, the linear correlation between $\alpha$ and mismatch shown in Example 2 was used.

More specifically, the data in Example 2 were fitted to a function $MM = m * \alpha + b$ using linear regression, MM being the mismatch value (PaCO$_2$ - EtCO$_2$). For determining the mismatch values from the actual measurement, the same formula was used by inserting the values obtained for the slope m and the intercept b, as well as the measured angle $\alpha$. PaCO$_2$ was then calculated by adding the mismatch value to EtCO$_2$ (PaCO$_2$ = EtCO$_2$ + MM).

**Example 2. Correlation between mismatch and capnogram shape**

In order to investigate the correlation between the mismatch and parameters relating to the capnogram shape, capnograms of 100 subjects were recorded and analyzed, essentially as described in Example 1. The study included 34 healthy subjects and 66 patients diagnosed with COPD.

In addition to the capnographic measurements, blood from each subject was collected and the $CO_2$ concentration was analyzed by blood gas analysis. The mismatch between $EtCO_2$ determined by capnography and the $PaCO_2$ determined from the blood samples was calculated. Figure 3A shows the correlation between the mismatch and the angle $\alpha$ between the expiratory upstroke 2 and the alveolar plateau 3. As can be seen from the figure, a strong correlation was observed.

Figure 3B shows the same correlation, wherein the datapoints from healthy subjects ("other") and the COPD patients are identified. As can be seen from this figure, in both cases a strong correlation between the angle $\alpha$ and the mismatch was observed.

## Example 3. Validation with human subjects

A validation study was carried out with 101 human subjects, 67 of whom were COPD patients. For each subject, $PaCO_2$ levels were determined by capnography and analyzed by the inventive method as described in Example 1. For comparison, the data obtained from the capnographic measurement were used without mismatch correction, by simply determining the $EtCO_2$ level. In addition, immediately following the capnographic measurement, blood was drawn from the subjects' earlobes to obtain capillary blood samples for blood gas analysis. The $CO_2$ levels obtained using capnography with or without the inventive method as well as using blood gas analysis were then compared. The following results were obtained:

- Capnographic measurement without mismatch correction ($EtCO_2$): approx. 28 % of the measurements were within +/- 5 mmHg of the results obtained from blood gas analysis;

- Capnographic measurement according to the invention (including mismatch correction as described in Example 1): approx. 71 % of the measurements were within +/- 5 mmHg of the results obtained from blood gas analysis.

Thus, as can be seen from these results, the $PaCO_2$ values obtained using the inventive method were much closer to the values determined by blood gas analysis than when only the $EtCO_2$ values were used (as is done with common capnometers or capnographs). The improvement in the accuracy was observed both for healthy subjects and COPD patients, but was particularly pronounced with sick patients.

## Example 4. Correction based on further parameters characterizing the shape of the $CO_2$ waveform signal

A master breathing curve was obtained as described in Example 1 and used as the $CO_2$ waveform signal for further analysis. Also, the $EtCO_2$ value was determined in the same way as described in Example 1. However, an alternative approach was used to analyze the shape of the $CO_2$ waveform signal.

First, the $CO_2$ waveform signal was fitted using cubic splines. The $CO_2$ waveform signal was described by the following mathematical function:

$$f(t; c_1, c_2, \tau, k) = c_2 - (c_2 - c_1)e^{-\frac{t-t_0}{\tau}} + k(t - t_0)$$

$$\frac{d}{dt} f(t; c_1, c_2, \tau, k) = \frac{(c_2 - c_1)}{\tau} e^{-\frac{t-t_0}{\tau}} + k$$

The parameters $c_1$ and $c_2$ are given with:

$$c_2 = c_1 + \tau(f'(t_0) - k)$$

$$f(t_0) = c_1$$

In the above formulas, t is the time, $t_0$ is time at the inflection point marking the start of the expiratory upstroke, k the constant of the linear term, and $\tau$ is the time constant of the exponential term. The parameters are determined by fitting this mathematical function to the Mastercurve beginning at $t_0$.

The parameter $\tau$ describes how stretched or compressed the $CO_2$ waveform signal is. The mismatch correction was carried out using this parameter $\tau$ and the linear term k.

Calculation of $PaCO_2$

The $PaCO_2$ level was calculated by applying the $CO_2$ mismatch correction factor based on the parameters $\tau$ and k to the obtained $EtCO_2$ value. For this purpose, a linear correlation between those factors and mismatch analog to that shown in Example 2 was used.

More specifically, the data in Example 4 were fitted to a function $MM = m_1 * \tau + m_2 * k + b$ using linear regression, MM being the mismatch value ($PaCO_2$ - $EtCO_2$). For determining the mismatch values from the actual measurement, the same formula was used by inserting the values obtained for the slopes $m_1$ and $m_2$ and the intercept b, as well as the factors $\tau$ and k. $PaCO_2$ was then calculated by adding the mismatch value to $EtCO_2$ ($PaCO_2 = EtCO_2 + MM$).

The data from the validation study in Example 3 were analyzed using the above mismatch correction instead of using the angle $\alpha$. The following results were obtained:

- Capnographic measurement without mismatch correction ($EtCO_2$): approx. 28 % of the measurements were within +/- 5 mmHg of the results obtained from blood gas analysis;

- Capnographic measurement according to the invention (including mismatch correction as described in Example 1): approx. 79 % of the measurements were within +/- 5 mmHg of the results obtained from blood gas analysis.

Thus, the above-described method for analyzing the shape of the $CO_2$ waveform signal led to ever higher accuracy than the analysis based on the angle $\alpha$.

**Claims**

1. A method for determining an arterial $CO_2$ concentration ($PaCO_2$) level in a subject, the method comprising the following steps:

   - measuring $CO_2$ concentration levels in exhaled breath from the subject over a period of time in order to obtain a capnography signal;
   - deriving a $CO_2$ waveform signal from the capnography signal;
   - determining an End-tidal $CO_2$ ($EtCO_2$) value from the $CO_2$ waveform signal;
   - analyzing a shape of the $CO_2$ waveform signal in order to determine a $CO_2$ mismatch correction factor;
   - applying the $CO_2$ mismatch correction factor to the $EtCO_2$ value in order to obtain the $PaCO_2$ level.

2. The method according to claim 1, wherein the $CO_2$ waveform signal comprises at least one expiratory upstroke (2) and one alveolar plateau (3), wherein the $CO_2$ mismatch correction factor is based on an angle ($\alpha$) between the expiratory upstroke (2) and the alveolar plateau (3).

3. The method according to claim 1, wherein the $CO_2$ waveform signal comprises at least one expiratory upstroke (2) and one alveolar plateau (3), wherein analyzing a shape of the $CO_2$ waveform signal comprises fitting a transition between the expiratory upstroke (2) and the alveolar plateau (3) to an exponential curve, wherein the $CO_2$ mismatch correction factor is based on a time constant ($\tau$) of the exponential curve.

4. The method according to any one of the preceding claims, wherein the $CO_2$ waveform signal corresponds to the $CO_2$ concentration as a function of time.

5. The method according to any one of the preceding claims, wherein the $CO_2$ waveform signal corresponds to the $CO_2$ concentration as a function of exhaled volume.

6. The method according to any one of the preceding claims, wherein the $CO_2$ concentration levels are measured over a period of time of between 10 seconds and 5 minutes.

7. The method according to any one of the preceding claims, wherein the $CO_2$ waveform signal corresponds to the average of at least two breathing cycles.

8. The method according to any one of the preceding claims, wherein the method does not comprise determining the concentration levels of other gases in exhaled breath, especially wherein the method does not comprise determining oxygen concentration levels.

9. The method according to any one of the preceding claims, wherein the subject is an asthma patient, a Chronic Obstructive Pulmonary Disease (COPD) patient, and/or a pulmonary fibrosis patient.

10. The method according to any one of the preceding claims, wherein the method is for monitoring mismatch values in the subject, wherein the determination of the $PaCO_2$ level is performed at least 4 times over a period of time of at least 2 weeks.

11. An apparatus for determining a $PaCO_2$ concentration level in a subject, the apparatus comprising:

   - at least one capnography sensor configured to measure $CO_2$ concentration levels in exhaled breath from the subject as a function of time;
   - a processor configured to carry out the following steps:

     ◦ obtaining from the capnography sensor a capnography signal comprising $CO_2$ concentration levels in exhaled breath from the subject over a period of time;
     ◦ deriving a $CO_2$ waveform signal from the capnography signal;
     ◦ determining an End-tidal $CO_2$ ($EtCO_2$) value from the $CO_2$ waveform signal;
     ◦ analyzing a shape of the $CO_2$ waveform signal in order to determine a $CO_2$ mismatch correction factor;
     ◦ applying the $CO_2$ mismatch correction factor to the $EtCO_2$ value in order to obtain the $PaCO_2$ level.

12. The apparatus according to claim 11, wherein the apparatus further comprises a flow sensor.

13. The apparatus according to any one of the preceding claims, wherein the apparatus further comprises a sample chamber for taking up exhaled breath from the subject, wherein the sample chamber is temperature-controlled.

14. The apparatus according to any one of the preceding claims, wherein the apparatus comprises a temperature sensor and/or a humidity sensor and wherein the processor is configured to correct the $CO_2$ concentration levels obtained from the capnography sensor based on the measured temperature and/or humidity.

15. The apparatus according to any one of the preceding claims, wherein the processor is configured to carry out the steps as defined in any one of claims 1 to 10.

Fig.1

Fig.2A

Fig.2B

Fig.2C

Fig.3A

Fig.3B

# EP 4 371 481 A1

**EUROPEAN SEARCH REPORT**

Application Number

EP 22 20 8583

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/244900 A1 (KREMEIER PETER [DE]) 12 August 2021 (2021-08-12) | 1,4-13, 15 | INV. A61B5/145 A61B5/083 |
| Y | * paragraph [0037] * | 14 | |
| X | US 5 632 281 A (RAYBURN DANIEL B [US]) 27 May 1997 (1997-05-27) | 1,2, 4-13,15 | |
| Y | * column 4, line 6 - column 9, line 65; claims 1,4; figures 1-6; table II * | 3,14 | |
| Y | US 2009/118633 A1 (JAFFE MICHAEL B [US] ET AL) 7 May 2009 (2009-05-07) * paragraphs [0045] - [0048]; figures 5,6,10 * | 3 | |
| Y | RASERA C C ET AL: "The effect of body temperature on the accuracy of arterial and end-tidal carbon dioxide measurement", MEASUREMENT, INSTITUTE OF MEASUREMENT AND CONTROL. LONDON, GB, vol. 44, no. 1, 1 January 2011 (2011-01-01), pages 60-64, XP027503371, ISSN: 0263-2241, DOI: 10.1016/J.MEASUREMENT.2010.09.026 [retrieved on 2010-11-17] * the whole document * | 14 | TECHNICAL FIELDS SEARCHED (IPC) A61B |
| X | US 2016/150997 A1 (COLMAN JOSHUA LEWIS [IL] ET AL) 2 June 2016 (2016-06-02) * the whole document * | 1,11 | |

-/--

The present search report has been drawn up for all claims

| Place of search | Date of completion of the search | Examiner |
|---|---|---|
| Berlin | 21 April 2023 | Kronberger, Raphael |

| Europäisches Patentamt European Patent Office Office européen des brevets | **EUROPEAN SEARCH REPORT** | Application Number EP 22 20 8583 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JAFFE MICHAEL B: "Using the features of the time and volumetric capnogram for classification and prediction", JOURNAL OF CLINICAL MONITORING AND COMPUTING, SPRINGER NETHERLANDS, DORDRECHT, vol. 31, no. 1, 18 January 2016 (2016-01-18), pages 19-41, XP036142400, ISSN: 1387-1307, DOI: 10.1007/S10877-016-9830-Z [retrieved on 2016-01-18] * Section 5.1 * ----- | 1,11 | |
| | | | TECHNICAL FIELDS SEARCHED (IPC) |

The present search report has been drawn up for all claims

| Place of search | Date of completion of the search | Examiner |
|---|---|---|
| Berlin | 21 April 2023 | Kronberger, Raphael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 20 8583

This annex lists the patent family members relating to the patent documents cited in the above-mentioned European search report.
The members are as contained in the European Patent Office EDP file on
The European Patent Office is in no way liable for these particulars which are merely given for the purpose of information.

21-04-2023

| Patent document cited in search report | | Publication date | Patent family member(s) | Publication date |
|---|---|---|---|---|
| US 2021244900 | A1 | 12-08-2021 | DE 102021000313 A1 | 12-08-2021 |
| | | | EP 3862040 A1 | 11-08-2021 |
| | | | US 2021244900 A1 | 12-08-2021 |
| US 5632281 | A | 27-05-1997 | NONE | |
| US 2009118633 | A1 | 07-05-2009 | NONE | |
| US 2016150997 | A1 | 02-06-2016 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2438855 A2